# EUROPÄISCHE PATENTSCHRIFT

(11) **EP 3 919 106 B1**
(45) Veröffentlichungstag und Bekanntmachung des Hinweises auf die Patenterteilung: **02.08.2023**
(21) Anmeldenummer: 21177030.0
(22) Anmeldetag: 01.06.2021
(51) Int. Cl.: A61M 16/04, A61F 13/02

(54) **TRACHEOSTOMAEPITHESE UND VERFAHREN ZUR HERSTELLUNG EINER TRACHEOSTOMAEPITHESE**
TRACHEOSTOMA EPITHESIS AND METHOD FOR PRODUCING A TRACHEOSTOMA EPITHESIS
ÉPITHÈSE DE TRACHÉOSTOMIE ET PROCÉDÉ DE FABRICATION D'UNE ÉPITHÈSE DE TRACHÉOSTOMIE

(30) Priorität: 03.06.2020 DE 102020114684
(43) Veröffentlichungstag der Anmeldung: 08.12.2021
(73) Patentinhaber: Andreas Fahl Medizintechnik-Vertrieb GmbH, 51149 Köln (DE)
(72) Erfinder: FAHL, Andreas, 51149 Köln (DE)
(74) Vertreter: Geskes, Christoph

(56) Entgegenhaltungen:
- EP-A1- 3 546 002
- WO-A1-91/05579
- WO-A1-94/19045
- WO-A1-2018/009118
- DE-A1-102010 048 316
- DE-A1-102010 049 895
- DE-U1-202008 017 105
- DE-U1-202012 013 326
- DE-U1-202013 010 194
- US-A1- 2013 192 604

## Beschreibung

Die Erfindung betrifft eine Tracheostomaepithese und ein Verfahren zur Herstellung einer Tracheostomaepithese.

Aufnahmen für Tracheostomahilfsmittel, wie beispielsweise Wärme-Feuchtigkeits-Austauscher sind aus dem Stand der Technik allgemein bekannt. Diese werden auf Pflaster aufgebracht, die über das Tracheostoma geklebt werden. So geht aus der DE 10 2013 001 910 A1 ein Tracheostomapflaster hervor, das auf der distalen Seite eine Aufnahme für ein Tracheostomahilfsmittel aufweist. WO 94/19045 A1 offenbart ein Sprechventil, welches mittels einer Basis über ein Tracheostoma anordenbar ist. DE 10 2010 048 316 A1 offenbart eine Beatmungseinrichtung zum Aufsetzen auf ein Tracheostoma. Diese weist ein planares, bewegliches, flexibles Flachteil mit einem zentralen Loch auf. DE 20 2013 010 194 U1 offenbart ein Sprechventil, das mittels eines Pflasters, ausgestaltet als dünne, planare, flexible Folie, über einem Tracheostoma angeordnet wird. DE 10 2010 049 895 A1 offenbart ein Haltepflaster für Trachealkanülen oder künstliche Nasen mit Sprechventil. Das Pflaster ist als dünne, planare flexible Folie ausgestaltet. WO 91/05579 A1 offenbart ein Tracheostomapflaster mit einem Adapter. Auf der proximalen Seite des Tracheostomapflasters kann ein Ring zum Absorbieren von Flüssigkeit angeordnet sein. EP 3 546 002 A1 offenbart eine Epithese, die mit unterschiedlichen Epithesenmaterialien gegossen werden kann, um unterschiedliche Färbungen und unterschiedliche Härten in verschiedenen Bereichen der Tracheostomaepithese zu generieren.

Nachteilig an diesen Pflastern ist, dass diese Einwegprodukte sind und Abfall produzieren. Weiterhin nachteilig ist, dass die Aufnahmen über dem Tracheostoma aufbauen und die Aufnahmen sowie die darin angeordneten Tracheostomahilfsmittel als unästhetisch und unkomfortabel wahrgenommen werden.

Aufgabe der Erfindung ist es daher die Nachteile aus dem Stand der Technik zu überkommen. Insbesondere ist die Aufgabe der Erfindung eine verbesserte Anordnung für Aufnahmen von Tracheostomahilfsmitteln zur Verfügung zu stellen. Insbesondere ist die Aufgabe der Erfindung Abfall bei der Versorgung von Tracheostomapatienten zu reduzieren. Insbesondere ist die Aufgabe der Erfindung Tracheostomapatienten eine ästhetische und komfortable Möglichkeit zu bieten, Tracheostomahilfsmittel zu verwenden.

Die Aufgabe wird erfindungsgemäß gelöst mittels einer Tracheostomaepithese zumindest umfassend einen Epithesenkörper und eine Aufnahmevorrichtung, wobei der Epithesenkörper eine Grundform mit einer distalen Grundfläche und einer proximalen Mantelfläche aufweist, wobei der Epithesenkörper eine Atemöffnung umfasst, die sich durchgängig von proximal nach distal erstreckt, wobei die Aufnahmevorrichtung einen Aufnahmezylinder und eine Abdeckplatte aufweist, wobei die Abdeckplatte auf der distalen Grundfläche des Epithesenkörpers angeordnet ist und wobei die Abdeckplatte mit dem Aufnahmezylinder verbunden ist.

Weiterhin wird die Aufgabe erfindungsgemäß gelöst mittels eines Verfahrens zur Herstellung einer Tracheostomaepithese nach einem oder mehreren der vorhergehenden Ansprüche, dadurch gekennzeichnet, dass diese im Mehr-Komponenten-Spritzguss hergestellt wird, wobei eine erste Komponente den Epithesenkörper bildet und eine zweite Komponente die Aufnahmevorrichtung bildet.

Weiterhin wird die Aufgabe erfindungsgemäß gelöst mittels eines Systems zumindest umfassend eine Tracheostomaepithese, ein Pflaster und ein Tracheostomahilfsmittel, wobei das Pflaster auf der Tracheostomaepithese aufbringbar ist und das Tracheostomahilfsmittel in eine Aufnahmevorrichtung der Tracheostomaepithese einbringbar ist.

Epithesen werden zum ästhetischen Ausgleich von Körperdefekten mittels eines körperfremden Materials wie beispielsweise Glas, Porzellan, Gummi, Metall oder Kunststoff verwendet. Bei einer Tracheotomie oder Laryngotomie gelingt es dem Chirurgen nicht immer ein rundes gut passendes Tracheostoma insbesondere in einem Halsprofil anzulegen, welches um das Tracheostoma relativ flach ist. Zudem können postoperative Heilungsstörungen und Tumorrezidive die Tracheostomaform ungünstig beeinflussen. Gegebenenfalls kann es dazu kommen, dass Kanülen nicht in das Tracheostoma passen und/oder Pflaster nicht auf den umgebenden Hautbereich des Tracheostomas haften. In diesen Fällen wird eine Tracheostomaepithese für den Patienten angefertigt. Insbesondere wenn die Haut um das Tracheostoma faltig ist und/oder Nischen aufweist und/oder das Tracheostoma ungünstig zwischen den Ansätzen des Musculus sternocleidomastoideus liegt, ist das luftdichte Abdichten zum Sprechen mit Standardhilfsmittel nicht zu erreichen. Zur Herstellung der Tracheostomaepithese sind individuelle Abformmittel, wie beispielsweise Gips oder eine Silikonabformmasse, bekannt. In einigen Fällen wird in die Epithese eine Standardkanüle integriert. Die aus dem Stand der Technik bekannten Epithesen weisen in der Regel eine Formgebung auf, die zumindest teilweise in die Trachea eindringt und insbesondere das Tracheostoma ungünstig verengt. Zur Befestigung von Tracheostomaepithesen wird üblicherweise ein Pflaster oder ein Patch verwendet, auf das eine Aufnahmevorrichtung befestigt sein kann, die zur Aufnahme von Tracheostomahilfsmitteln geeignet ist.

Die vorgeschlagene Tracheostomaepithese kann beispielsweise mittels eines Pflasters am Hals eines Benutzers befestigt werden. Hierzu wird das Pflaster auf eine erste beziehungsweise distale Seite der Epithese geklebt und überlappende Bereiche am Hals des Patienten befestigt. Bevorzugt weist das Pflaster ein Loch auf, das über der Aufnahmevorrichtung angeordnet wird, so dass ein Zugang zur Aufnahmevorrichtung frei bleibt und der Benutzer atmen kann. Das Pflaster selber braucht nun keine Aufnahme mehr aufzuweisen, so dass die Abfallmenge reduziert werden kann. Die Tracheostomaepithese ist bevorzugt mehrfach verwendbar.

Die Tracheostomaepithese weist einen Epithesenkörper auf. In einer weiteren Ausgestaltung ist vorgesehen, dass der Epithesenkörper eine vorgefertigte Formgebung aufweist. Der Epithesenkörper weist eine Grundform mit einer distalen Grundfläche und einer proximalen Mantelfläche auf. Grundfläche und Mantelfläche sind auf gegenüberliegenden Seiten ausgebildet. Vorzugsweise ist der Epithesenkörper im Wesentlichen kegelstumpfförmig ausgestaltet, wobei die Grundfläche als eine Kegel-Grundfläche und die Mantelfläche als eine KegelMantelfläche ausgestaltet ist. In einer weiteren Ausgestaltung ist der Epithesenkörper im Wesentlichen kalottenförmig ausgestaltet, wobei die Grundfläche als insbesondere kreisförmige Kalotten-Grundfläche und die Mantelfläche als Kuppel ausgebildet ist. Bevorzugt grenzen Grundfläche und Mantelfläche an einer umlaufenden Kante aneinander an. Bevorzugt verjüngt sich ein Durchmesser des Epithesenkörpers von distal nach proximal beziehungsweise in eine Richtung von der Grundfläche weg. Weiter bevorzugt weist der Epithesenkörper in einer Aufsicht von distal eine rechteckige, eine ovale, eine runde oder eine sonstige Formgebung auf. Insbesondere können Ecken abgerundet sein und/oder eine oder mehrere der vorgenannten Formgebungen zu weiteren Formgebungen miteinander kombiniert werden, insbesondere können zwei in etwa kreisförmige Ausgestaltungen mit unterschiedlichen Durchmessern zu einer Form des Epithesenkörpers beziehungsweise der Grundfläche verbunden sein. Bevorzugt ist der Epithesenkörper kegelförmig ausgestaltet, wobei die erste beziehungsweise distale Seite eine Grundfläche einer Kegelform und weiter bevorzugt eine zweite beziehungsweise proximale Seite einer Mantelfläche der Kegelform entspricht. Insbesondere ist die distale Seite rund, elliptisch, rechteckig, insbesondere mit abgerundeten Kanten oder in einer anderen beliebigen Form ausgebildet. In einer Ausgestaltung ist vorgesehen, dass die Mantelfläche bevorzugt im Wesentlichen kegelmantelförmig und/oder im Wesentlichen eine Negativform eines ein Tracheostoma umgebenen Hautbereiches ist. Weiterhin ist bevorzugt vorgesehen, dass eine Hochachse des Kegels beziehungsweise des Epithesenkörpers mit einer Längsachse der Atemöffnung übereinstimmt. Die Mantelfläche kann bevorzugt unterschiedliche Neigungen aufweisen.

Im Sinne der Erfindung ist ein Kegel beziehungsweise ein Konus ein geometrischer Körper, bei dem alle Punkte einer Grundfläche gradlinig mit einer Spitze beziehungsweise einem Punkt einer Ebene, in der nicht die Grundfläche angeordnet ist, verbunden sind. Die Grundfläche kann kreisförmig, rechteckig, oval oder jede beliebige andere Form, insbesondere mit abgerundeten Ecken, aufweisen. Weiterhin weist im Sinne der Erfindung eine konische Ausnehmung bevorzugt eine kreiskegelförmige Ausgestaltung auf. Unter einem Kegel oder einem Konus kann im Sinne der Erfindung auch ein Kegelstumpf verstanden werden. Insbesondere ist vorgesehen, dass die konische Ausnehmung sich derart durch den kegelförmigen Epithesenkörper erstreckt, dass der Epithesenkörper als Kegelstumpf ausgebildet ist. Weiterhin weist bevorzugt die konische Ausnehmung eine theoretische Konusspitze auf, die außerhalb des Epithesenkörpers liegt. Somit ist eine Ausnehmungswandungsoberfläche, die die Ausnehmung innerhalb des Epithesenkörpers begrenzt, kegelstumpfförmig beziehungsweise weist die Form eines Mantels eines Konusstumpfes auf. In einer Ausgestaltung ist eine Längsachse der Atemöffnung vorzugsweise mit der Hochachse des kegelförmigen Epithesenkörpers, die ein Lot von einer Spitze auf die Grundfläche der Kegelform ist, kongruent.

In einer weiteren Ausgestaltung ist der Epithesenkörper individuell an den Benutzer angepasst, insbesondere an ein Tracheostoma sowie an den das Tracheostoma umgebenden Bereich. Bevorzugt erfolgt eine Anpassung des Epithesenkörpers mittels eines 3D-Scans oder eines Abdrucks, der von dem das Tracheostoma umgebenden Bereich erzeugt wird. Weiter bevorzugt weist der Epithesenkörper, bevorzugt in einer kegelförmigen Ausführungsform einen Öffnungswinkel der Mantelfläche von etwa 120° bis etwa 160°, bevorzugt etwa 140° auf, der, wie oben erwähnt, unterschiedlich sein kann, insbesondere auf in etwa gegenüberliegenden Mantelflächenbereichen.

Wenn im Rahmen der Beschreibung Richtungsangaben verwendet werden, so sind diese in Bezug auf den üblichen Gebrauch der Tracheostomaepithese zu verstehen. Richtungsbezeichnungen werden wie in der Anatomie üblich verwendet. Unter dem Begriff "distal" ist im Sinne der vorliegenden Erfindung zu verstehen, dass ein Merkmal in eine Richtung fern oder auch abgewandt einer Hautoberfläche eines Benutzers, der insbesondere die Tracheostomaepithese trägt, anordenbar ist. So ist beispielsweise die Grundfläche des Epithesenkörpers von einem menschlichem Körper, insbesondere einem Tracheostoma, wegweisend anordenbar ausgebildet. Unter "proximal" ist im Sinne der vorliegenden Erfindung zu verstehen, dass ein Merkmal nah oder auch zugewandt einer Hautoberfläche des Benutzers, der insbesondere die Tracheostomaepithese trägt anordenbar ist. So ist beispielsweise die Mantelfläche des Epithesenkörpers einem menschlichem Körper, insbesondere einem Tracheostoma, zuordenbar beziehungsweise zuwendbar ausgebildet.

Der Begriff "im Wesentlichen" gibt einen Toleranzbereich an, der für den Fachmann unter wirtschaftlichen und technischen Gesichtspunkten zu vertreten ist, so dass das entsprechende Merkmal noch als solches zu erkennen oder verwirklicht ist.

In einer Ausgestaltung ist vorgesehen, dass der Epithesenkörper zumindest aus einem Material ist ausgewählt aus einer Gruppe umfassend Silikon und/oder selbstklebende Kunststoffe. Bevorzugt ist das Material des Epithesenkörpers ein inhärent selbstklebendes Material, beispielsweise selbstklebendes Silikon.

Werden in Rahmen der Erfindung beispielhafte Aufzählungen aufgeführt, so sind diese als nicht abschließend zu betrachten. Vielmehr sind beispielhafte Aufzählungen Hinweise auf weitere dem allgemeinen Fachwissen entnehmbare Ausführungen.

Der Epithesenkörper weist zudem eine Atemöffnung auf, die sich in einer Ausgestaltung von der ersten Seite zur zweiten Seite beziehungsweise von distal nach proximal erstreckt. Die Atemöffnung ist in einer Ausgestaltung zylindrisch ausgestaltet. In einer weiteren Ausgestaltung ist die Atemöffnung kegelförmig oder kegelstumpfförmig ausgestaltet. Insbesondere weitet sich die Atemöffnung in einer Ausgestaltung nach distal oder proximal auf. Besonders bevorzugt weist die Atemöffnung einen Durchmesser auf, der die Aufnahmevorrichtung aufnehmen kann. Weiter bevorzugt weist die Atemöffnung einen Innendurchmesser auf, der im Wesentlichen gleich ist mit einem Außendurchmesser einer Aufnahmevorrichtung.

Ein Öffnungswinkel des Konus bestimmt sich insbesondere durch einen distalen Öffnungsdurchmesser der Atemöffnung und einen proximalen Öffnungsdurchmesser der Atemöffnung sowie der Dicke des Epithesenkörpers. Bevorzugt ist der distale Öffnungsdurchmesser etwa 5 mm bis etwa 22 mm, weiter bevorzugt etwa 10 mm bis etwa 22 mm, weiter bevorzugt etwa 15 mm, weiter bevorzugt etwa 22 mm. In einer Ausgestaltung ist der proximale Öffnungsdurchmesser etwa 15 mm bis etwa 40 mm, bevorzugt etwa 15 mm bis etwa 30 mm, weiter bevorzugt etwa 15 mm, weiter bevorzugt etwa 22 mm. Ist der Aufnahmezylinder in der Atemöffnung angeordnet, ist bevorzugt der distale Öffnungsdurchmesser und/oder der proximale Öffnungsdurchmesser durch einen Außendurchmesser des Aufnahmezylinders bestimmt. Eine Höhe des Epithesenkörpers von der ersten Seite zur zweiten Seite beziehungsweise von proximal nach distal ist in einer Ausgestaltung etwa 2 mm bis etwa 50 mm, bevorzugt etwa 2 mm bis etwa 25 mm, weiter bevorzugt etwa 8 mm bis etwa 25 mm.

Die Aufnahmevorrichtung weist einen Aufnahmezylinder und eine Abdeckplatte auf. Bevorzugt ist der Aufnahmezylinder ein Hohlzylinder. Weiter bevorzugt weist der Aufnahmezylinder einen Respirationsschutz auf, der bevorzugt dem proximalen Ende des Aufnahmezylinders zugeordnet ist. Der Respirationsschutz ist beispielsweise als zumindest ein Steg, ein Sieb und/oder ein Gitter ausgestaltet. Bevorzugt ist der Aufnahmezylinder zur Aufnahme von Tracheostomahilfsmitteln ausgestaltet. Tracheostomahilfsmittel können beispielsweise ein Sprechventil und/oder ein Wärme-Feuchtigkeits-Austauscher sein. Ein Tracheostomahilfsmittel kann auch eine Trachealkanüle sein. In einer Ausgestaltung ist vorgesehen, dass der Aufnahmezylinder eine Trachealkanüle aufnehmen kann. Beispielsweise ist ein Kanülenrohr der Trachealkanüle durch den Aufnahmezylindersteckbar. In einer weiteren Ausgestaltung ist die Trachealkanüle mittels des Aufnahmezylinders befestigbar. In einer Ausgestaltung ist vorgesehen, dass der Aufnahmezylinder zumindest ein Rastmittel zum Halten zumindest eines Tracheostomahilfsmittels aufweist. Das Rastmittel ist beispielsweise ausgestaltet als zumindest teilweise umlaufender Wulst, als zumindest teilweise umlaufende Kerbe, als eine Anzahl von sich in den Aufnahmezylinder erstreckenden Erhebungen und/oder als Teil eines Bajonettverschlusses.

Die Abdeckplatte stabilisiert vorteilhafter Weise den Aufnahmezylinder in dem relativ weichen Material des Epithesenkörpers. Hierdurch lässt sich das Tracheostomahilfsmittel leicht in den Aufnahmezylinder einbringen und entfernen. Auch wird ein zu weites Eindrücken oder Herausziehen des Aufnahmezylinders in den beziehungsweise aus dem Epithesenkörper bei der Handhabung beispielsweise eines Tracheostomahilfsmittels verhindert. Weiterhin vorteilhaft wird der Aufnahmezylinder mit seiner sich von proximal nach distal erstreckenden Längsachse durch die Abdeckplatte stabilisiert. Bevorzugt wird ein Kippen des Aufnahmezylinders, insbesondere bei einer Handhabung eines Tracheostomahilfsmittels in der Aufnahmevorrichtung, verhindert. Die Abdeckplatte umgibt in einer Ausgestaltung den Aufnahmezylinder zumindest teilweise. Bevorzugt umgibt die Abdeckplatte den Aufnahmezylinder vollständig. In einer Ausführungsform ist vorgesehen, dass die Abdeckplatte sich radial von dem Aufnahmezylinder in Richtung eines Außenumfanges des Epithesenkörpers erstreckt. Bevorzugt bedeckt die Abdeckplatte die Grundfläche. Bevorzugt bedeckt die Abdeckplatte die Grundfläche des Epithesenkörpers vollflächig. Dies ist insbesondere dann von Vorteil, wenn der Epithesenkörper ein inhärent klebendes Material aufweist, so dass die Tracheostomaepithese leicht gehandhabt werden kann, ohne dass diese beispielsweise an der Hand oder Kleidungsstücken des Benutzers hängen bleibt. Die Abdeckplatte kann aber auch die Grundfläche des Epithesenkörpers nur teilweise bedecken. Weiterhin vorteilhaft weist die Abdeckplatte ein Material auf, auf dem ein Pflaster aufklebbar und vorzugsweise rückstandsfrei wieder ablösbar ist. In einer weiteren Ausgestaltung ist vorgesehen, dass die Abdeckplatte zumindest teilweise gitterförmig oder siebförmig ausgestaltet ist.

Die Abdeckplatte ist bevorzugt mit dem Aufnahmezylinder verbunden. In einer Ausgestaltung ist vorgesehen, dass der Aufnahmezylinder und die Abdeckplatte materialeinheitlich oder materialverbunden sind. In einer weiteren Ausführungsform ist vorgesehen, dass die Abdeckplatte und der Aufnahmezylinder geklebt, geklemmt und/oder formschlüssig verbunden, beispielsweise gerastet, sind.

Bevorzugt weist die Aufnahmevorrichtung ein von dem Epithesenkörper unterschiedliches Material auf. Weiter bevorzugt weist die Aufnahmevorrichtung ein nicht inhärent klebendes Material auf. Weiter bevorzugt weist die Aufnahmevorrichtung ein rigideres Material als der Epithesenkörper auf. In einer Ausgestaltung weist der Epithesenkörper eine Härte von etwa SHORE-OO 5 bis etwa SHORE-OO 50, bevorzugt etwa SHORE-OO 10 bis etwa SHORE-OO 30 auf. In einer Ausgestaltung ist vorgesehen, dass die Aufnahmevorrichtung, respektive der Aufnahmezylinder und/oder die Abdeckplatte, eine Härte von etwa SHORE-A 50 bis etwa SHORE-A 100, bevorzugt etwa SHORE-A 50 bis etwa SHORE-A 80 aufweist. SHORE-Härten im Sinne der Erfindung sind nach dem Standard ASTM D2240-00, Standard Test Method for Rubber Property-Durometer Hardness, ASTM International, West Conshohocken, PA, Version aus 2002, zu ermitteln.

Besonders bevorzugt weist die Aufnahmevorrichtung eine höhere Druckfestigkeit auf als der Epithesenkörper. In einer Ausgestaltung ist vorgesehen, dass die Aufnahmevorrichtung zumindest ein Material aufweist ausgewählt aus einer Gruppe umfassend thermoplastische Elastomere. Insbesondere ist das Material der Aufnahmevorrichtung nicht klebend ausgestaltet. In einer weiteren bevorzugten Ausgestaltung werden Epithesenkörper und Aufnahmevorrichtung in einem Mehr-Komponenten-Spritzgussverfahren hergestellt. Vorteilhafterweise werden durch den Mehr-Komponente-Spritzguss der Epithesenkörper und die Aufnahmevorrichtung unlösbar miteinander verbunden. Weiterhin kann durch den Mehr-Komponente-Spritzguss die Tracheostomaepithese schnell und günstig hergestellt werden.

In einer Ausgestaltung ist vorgesehen, dass die Abdeckplatte an einer proximalen Seite des Aufnahmezylinders angeordnet ist. Durch die proximale Anordnung steht der Aufnahmezylinder über den Epithesenkörper hervor. Diese Ausführungsform hat den Vorteil, dass die Aufnahmevorrichtung leicht zugänglich und beim Einbringen und Entfernen des Tracheostomahilfsmittels vom Benutzer gegriffen werden kann. In einer weiteren Ausgestaltung ist vorgesehen, dass die Abdeckplatte an einer distalen Seite des Aufnahmezylinders angeordnet ist. Durch die distale Anordnung der Abdeckplatte zum Aufnahmezylinder ist der Aufnahmezylinder im Wesentlichen in dem Epithesenkörper beziehungsweise in der Atemöffnung angeordnet oder versenkt.

Grundsätzlich erstreckt sich bevorzugt im Sinne der vorliegenden Anmeldung der Aufnahmezylinder unmittelbar ausgehend von der Abdeckplatte in die Atemöffnung des Epithesenkörpers hinein. Diese Ausgestaltung hat den Vorteil, dass die Tracheostomaepithese sehr flach ausgeführt ist und somit optisch nicht so sehr hervortritt, wie ein aus dem Stand der Technik bekanntes Tracheostomapflaster. Selbst wenn ein Tracheostomahilfsmittel in die Aufnahmevorrichtung eingesetzt ist, ist das Erscheinungsbild eher dezent, das Hilfsmittel fällt nicht so sehr in das Auge des Betrachters, als wenn der Aufnahmezylinder vorsteht. Auch ist durch den versenkten Aufnahmezylinder der Tragekomfort erhöht, da der Aufnahmezylinder beziehungsweise das Tracheostomahilfsmittel beispielsweise nicht an einem Kleidungsstück hängen bleiben kann. In einer weiteren Ausgestaltung ist vorgesehen, dass die Abdeckplatte an einer beliebigen Höhe von proximal nach distal am Aufnahmezylinder angeordnet ist. Insbesondere ist die Abdeckplatte etwa auf halber Höhe an dem Aufnahmezylinder angeordnet. Weiterhin bevorzugt ist vorgesehen, dass ein Rand des Aufnahmezylinders von etwa 1 mm bis etwa 10 mm, weiter bevorzugt etwa 1 mm bis etwa 5 mm, über die Abdeckplatte nach distal hervorragt, insbesondere bei einer Anordnung der Abdeckplatte an einem im Wesentlichen distalen Abschnitt am Aufnahmezylinder. Insbesondere ist in einer Ausgestaltung vorgesehen, dass der Aufnahmezylinder zumindest teilweise in der Atemöffnung angeordnet ist. Auf diese Weise können die Vorteile der vorgenannten Ausführungsformen zumindest teilweise genutzt werden.

Im Sinne der Erfindung ist unter der Begriff 'unmittelbar' räumlich so zu verstehen, dass zwei Teile direkt aneinander angrenzen und insbesondere miteinander klebend verbunden oder materialverbunden sind. Beispielsweise können Abdeckplatte und Aufnahmezylinder entweder in einem Mehr-Komponenten-Spritzgussverfahren gefertigt, miteinander verklebt oder in einem Stück gefertigt sein und grenzen somit unmittelbar aneinander an.

In einer ersten beispielhaften Ausgestaltung ist vorgesehen, dass die Tracheostomaepithese einen Epithesenkörper und eine Aufnahmevorrichtung aufweist. Die Aufnahmevorrichtung weist einen Aufnahmezylinder und eine Abdeckplatte auf, die miteinander verbunden, insbesondere materialverbunden, sind. Die Abdeckplatte ist einer im Wesentlichen proximalen Seite des Aufnahmezylinders zugeordnet, wobei der Aufnahmezylinder nach distal über die Abdeckplatte hervorsteht. Weiterhin weist der Aufnahmezylinder ein Rastmittel auf, das in der gezeigten Ausgestaltung als innen umlaufender Wulst ausgestaltet ist.

Die Abdeckplatte ist bevorzugt vollflächig auf der Grundfläche des Epithesenkörpers angeordnet. Besonders bevorzugt ist die Abdeckplatte auf der Grundfläche des Epithesenkörpers aufgeklebt oder mittels Mehr-Komponenten-Spritzguss mit dem Epithesenkörper verbunden. Die Abdeckplatte ist in diesem Beispiel der proximalen Seite des Aufnahmezylinders zugeordnet. Insbesondere ist die Abdeckplatte im Bereich eines proximalen Endes des Aufnahmezylinders angeordnet. Die Abdeckplatte verdeckt den Aufnahmezylinder nicht, sondern erstreckt sich um diesen herum beziehungsweise geht in einer Ausführung insbesondere materialeinheitlich in diesen über. Die Grundplatte der Aufnahmevorrichtung ist beispielsweise radial vom Rand des Epithesenkörpers beziehungsweise vom radial äußeren Rand der Abdeckplatte zum Aufnahmezylinder hin dicker werdend ausgestaltet. Durch diese Ausgestaltung der Abdeckplatte ist trotz des relativ zur Aufnahmevorrichtung sehr weich ausgestalteten Epithesenkörpers eine Stabilität des Aufnahmezylinders gewährleistet. Beispielsweise ist der Epithesenkörper aus einem bevorzugt selbstklebenden Silikon und die Aufnahmevorrichtung aus einem thermoplastischen Elastomer gefertigt.

Der Epithesenkörper ist beispielsweise kegelstumpfförmig ausgestaltet, wobei die Grundfläche proximal und die Mantelfläche distal angeordnet ist. Der Öffnungswinkel der Mantelfläche beträgt beispielsweise etwa 140° und kann unterschiedlich in insbesondere in etwa gegenüberliegenden Mantelflächenbereichen ausgebildet sein. Die Atemöffnung ist beispielsweise zylindrisch ausgestaltet. Wie oben erwähnt, kann die Atemöffnung auch in weiteren Ausführungsformen konusförmig, insbesondere nach proximal öffnend, ausgestaltet sein.

In einer zweiten beispielhaften Ausgestaltung ist alternativ zu der ersten beschriebenen Ausgestaltung die Abdeckplatte einer distalen Seite des Aufnahmezylinders zugeordnet. Insbesondere ist die Abdeckplatte im Bereich eines distalen Endes des Aufnahmezylinders angeordnet. Die Abdeckplatte verdeckt den Aufnahmezylinder beziehungsweise die Atemöffnung nicht, sondern erstreckt sich um diesen herum beziehungsweise geht in einer Ausführung insbesondere materialeinheitlich in diesen über. Der Aufnahmezylinder erstreckt sich unmittelbar ausgehend von der Abdeckplatte nach proximal in den Epithesenkörper hinein. Somit ist der Aufnahmezylinder ist im Wesentlichen im Epithesenkörper versenkt beziehungsweise innerhalb diesem angeordnet. Bevorzugt ist der Aufnahmezylinder im Wesentlichen in der Atemöffnung des Epithesenkörpers angeordnet. In der beispielhaften Ausgestaltung grenzt zumindest ein Teil einer Außen-Umfangsfläche des Aufnahmezylinders im Wesentlichen an zumindest einen Teil einer Innen-Umfangsfläche der Atemöffnung, beispielsweise ist der Epithesenkörper zumindest teilweise um den Aufnahmezylinder umspritzt. In einer Ausgestaltung kann der Aufnahmezylinder teilweise proximal und/oder distal aus dem Epithesenkörper ragen, insbesondere ragt ein proximaler Randbereich des Aufnahmezylinders proximal aus der Atemöffnung respektive dem Epithesenkörper heraus. Bevorzugt ist in einer Ausgestaltung vorgesehen, dass ein Randbereich des Aufnahmezylinders über die Abdeckplatte insbesondere nach distal herausragt, bevorzugt etwa 1 mm bis etwa 5 mm weit. Die Abdeckplatte erstreckt sich beispielhaft vollflächig auf der distalen Seite des Epithesenkörpers von der distalen Außenkante des Aufnahmezylinders in Richtung eines Außenumfanges des Epithesenkörpers. Bevorzugt grenzt die Abdeckplatte unmittelbar an den Außenumfang des Aufnahmezylinders an, wobei diese mit der Abdeckplatte bevorzugt verklebt oder materialverbunden ist. Auf diese Weise ist der Aufnahmezylinder in der Atemöffnung angeordnet und erstreckt sich beispielhaft zumindest über die vollständige Höhe des Epithesenkörpers durch diese. Weiter bevorzugt erstreckt sich der Aufnahmezylinder in einer Ausgestaltung nicht vollständig durch die Atemöffnung, vorzugsweise nur etwa ein Drittel bis etwa auf die Hälfte der Höhe des Epithesenkörpers. Auf diese Weise ist die Tracheostomaepithese, insbesondere der Epithesenkörper, besser an das Tracheostoma und den umgebenden Bereich anpassbar.

Die Tracheostomaepithese beider beispielhafter Ausgestaltungen kann beispielhaft über beziehungsweise distal einem Tracheostoma eines Patienten angeordnet werden. Insbesondere wird mittels der Tracheostomaepithese ein um das Tracheostoma befindliches eingefallenes Gewebe optisch aufgefüllt. Da der Epithesenkörper bevorzugt ein inhärent selbstklebendes Silikon aufweist, wird die Epithese am Tracheostoma umgebenden Gewebe vorfixiert, so dass der Benutzer in Ruhe das Pflaster auf der Tracheostomaepithese aufbringen und am Hals befestigen kann. Hiernach kann das Tracheostomahilfsmittel, das beispielhaft als Sprechventil ausgestaltet ist, in die Aufnahmevorrichtung eingebracht werden. Zum Entfernen der Tracheostomaepithese wird das Pflaster von der Abdeckplatte abgezogen und die Epithese von dem das Tracheostoma umgebenden Gewebe gelöst. Vorzugsweise ist eine Adhäsion des Epithesenkörpers derart, dass eine Ablösung desselben von der Haut des Benutzers schmerzfrei erfolgen kann, und insbesondere auch derart, dass bei einem Abziehen des Pflasters die Tracheostomaepithese am Pflaster verbleibt.

In einer Ausgestaltung ist vorgesehen, dass wenn der Benutzer die Tracheostomaepithese in Kombination mit einem Pflaster ohne Silikonkleber nutzt, sich das Pflaster ohne Probleme lösen lässt, so dass die Epithese im Tracheostoma verbleibt. In einer weiteren Ausgestaltung ist vorgesehen, dass ein Kleber, beispielsweise ein Silikonkleber, zur Verklebung mit dem Pflaster und/oder der Haut des Benutzers verwendet wird.

Weiterhin wird ein System zumindest umfassend eine Tracheostomaepithese wie vorstehend beschrieben, ein Pflaster und ein Tracheostomahilfsmittel vorgeschlagen, wobei das Pflaster auf der Tracheostomaepithese aufbringbar ist und das Tracheostomahilfsmittel in eine Aufnahmevorrichtung der Tracheostomaepithese einbringbar ist. Das Pflaster weist in einer bevorzugten Ausgestaltung ein Loch auf, das zumindest einen Durchmesser aufweist, der gleich oder größer einem Durchmesser einer Atemöffnung des Epithesenkörpers ist. In einer Ausgestaltung ragt das Pflaster über den Epithesenkörper, weiter bevorzugt über die Abdeckplatte hinaus, die weiter bevorzugt vollflächig und insbesondere unmittelbar auf dem Epithesenkörper angeordnet ist, so dass mittels des Pflasters die Epithese beispielsweise an einem Hals eines Benutzers befestigt werden kann.

Wie oben beschrieben kann der Epithesenkörper beispielsweise aus einem Silikon, bevorzugt inhärent selbstklebenden Silikon, gefertigt und die Aufnahmevorrichtung ein rigideres Material als der Epithesenkörper aufweisen, beispielsweise ein thermoplastisches Elastomer. Gemäß einer Ausführung weist die Aufnahmevorrichtung, weiter bevorzugt die insbesondere vollflächig und bevorzugt unmittelbar auf dem Epithesenkörper angeordnete, beispielsweise aufgeklebte oder im Mehr-Komponenten-Spritzguss aufgebrachte, Abdeckplatte eine Materialoberfläche auf, auf der das Pflaster klebend hält und insbesondere rückstandsfrei lösbar ist. Bevorzugt ist die Adhäsion des Epithesenkörpers auf der Haut des Benutzers schwächer als diejenige des jeweils verwendeten Pflasters. Wenn das Pflaster stärker an der Tracheostomaepithese hält als die Tracheostomaepithese an der Haut des Benutzers, kann beim Entfernen des Pflasters die Tracheostomaepithese in einem Schritt von dem das Tracheostoma umgebenden Gewebe mit gelöst werden. Die Tracheostomaepithese bleibt somit am Pflaster hängen. Hiernach kann zur Reinigung der Tracheostomaepithese das Pflaster bequem von der Tracheostomaepithese gelöst werden.

Beispielsweise ist das Tracheostomahilfsmittel ein Wärme-Feuchtigkeitsaustauscher, ein Sprechventil oder ein sonstiges Hilfsmittel für Tracheotomierte und/oder Laryngektomierte. Bevorzugt nimmt die Aufnahmevorrichtung das Tracheostomahilfsmittel auf. Weiter bevorzugt ist das Tracheostomahilfsmittel am oder im Aufnahmezylinder bevorzugt wieder-lösbar befestigbar, beispielsweise einrastbar.

Weitere vorteilhafte Ausgestaltungen gehen aus den nachfolgenden Zeichnungen hervor. Die dort dargestellten Weiterbildungen sind jedoch nicht beschränkend auszulegen, vielmehr können die dort beschriebenen Merkmale untereinander und mit den oben beschriebenen Merkmalen zu weiteren Ausgestaltungen kombiniert werden. Des Weiteren sei darauf verwiesen, dass die in der Figurenbeschreibung angegebenen Bezugszeichen den Schutzbereich der vorliegenden Erfindung nicht beschränken, sondern lediglich auf die in den Figuren gezeigten Ausführungsbeispiele verweisen. Gleiche Teile oder Teile mit gleicher Funktion weisen im Folgenden die gleichen Bezugszeichen auf. Es zeigen:
Fig. 1 eine Tracheostomaepithese mit distal zur Abdeckplatte angeordneten Aufnahmezylinder in einer perspektivischen Ansicht;
Fig. 2 die Tracheostomaepithese aus Fig. 1 in einer Seitenansicht;
Fig. 3 ein System umfassend die Tracheostomaepithese aus Fig. 1, ein Pflaster und ein Tracheostomahilfsmittel in einer Explosionsdarstellung;
Fig. 4 eine Tracheostomaepithese mit proximal zur Abdeckplatte angeordneten Aufnahmezylinder in einer perspektivischen Ansicht;
Fig. 5 die Tracheostomaepithese aus Fig. 4 in einer Seitenansicht;
Fig. 6 ein System umfassend die Tracheostomaepithese aus Fig. 4, ein Pflaster und ein Tracheostomahilfsmittel in einer Explosionsdarstellung; und
Fig. 7 die Tracheostomaepithese aus Fig. 4 mit einer Trachealkanüle.

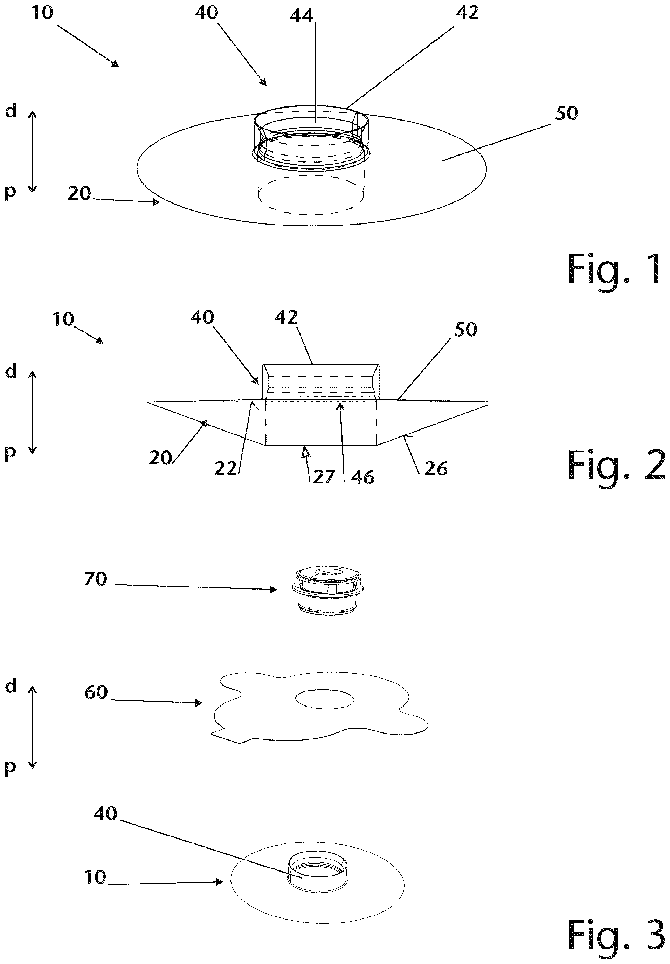

Fig. 1 zeigt eine Tracheostomaepithese 10 mit einem Epithesenkörper 20 und einer Aufnahmevorrichtung 40. Die Aufnahmevorrichtung 40 weist einen Aufnahmezylinder 42 und eine Abdeckplatte 50 auf, die miteinander verbunden, insbesondere materialverbunden, sind. Die Abdeckplatte 50 ist proximal p zum Aufnahmezylinder 42 angeordnet, wobei der Aufnahmezylinder nach distal über die Abdeckplatte hervorsteht. Weiterhin weist der Aufnahmezylinder 42 ein Rastmittel 44 auf, das in der gezeigten Ausgestaltung als innen umlaufender Wulst ausgestaltet ist.

Fig. 2 zeigt die Tracheostomaepithese 10 aus Fig. 1 in einer Seitenansicht. Die Abdeckplatte 50 ist insbesondere vollflächig auf der Grundfläche 22 der des Epithesenkörpers 20 angeordnet. Die Abdeckplatte 50 ist zudem der proximalen Seite 46 des Aufnahmezylinders 42 zugeordnet. Die Grundplatte 50 der Aufnahmevorrichtung 40 ist radial zum Aufnahmezylinder 42 hin dicker werdend ausgestaltet, insbesondere um eine Stabilität für den Aufnahmezylinder zu gewährleisten, da der Epithesenkörper 20 aus relativ zur Aufnahmevorrichtung 40 sehr weich ausgestaltet ist. Beispielsweise weist der Epithesenkörper 20 ein bevorzugt selbstklebendes Silikon auf und die Aufnahmevorrichtung 40 ein Thermoplastisches Elastomer auf.

Der Epithesenkörper 20 ist kegelstumpfförmig ausgestaltet, wobei die Grundfläche 22 proximal p und die Mantelfläche 26 distal d angeordnet ist. Eine Atemöffnung 27, deren Wandung gestrichelt im Epithesenkörper 20 eingezeichnet ist, ist zylindrisch ausgestaltet. Wie in der allgemeinen Beschreibung erwähnt, kann die Atemöffnung auch in weiteren Ausführungsformen konusförmig, insbesondere nach proximal p öffnend ausgestaltet sein.

Fig. 3 zeigt ein System umfassend die Tracheostomaepithese 10 aus Fig. 1 und 2 sowie eine Pflasters 60 und ein Tracheostomahilfsmittels 70 in einer Explosionsdarstellung. Die Tracheostomaepithese 10 wird über beziehungsweise distal einem Tracheostoma eines Patienten angeordnet. Insbesondere wird mittels der Tracheostomaepithese 10 ein um das Tracheostoma befindliches eingefallenes Gewebe optisch aufgefüllt. Da der Epithesenkörper 20 bevorzugt ein inhärent selbstklebendes Silikon aufweist, wird die Epithese am Tracheostoma umgebenden Gewebe vorfixiert, so dass der Benutzer in Ruhe das Pflaster 60 auf der Tracheostomaepithese 10 aufbringen und am Hals befestigen kann. Hiernach kann das Tracheostomahilfsmittel 70, das beispielhaft als Sprechventil ausgestaltet ist, in die Aufnahmevorrichtung 40 eingebracht werden.

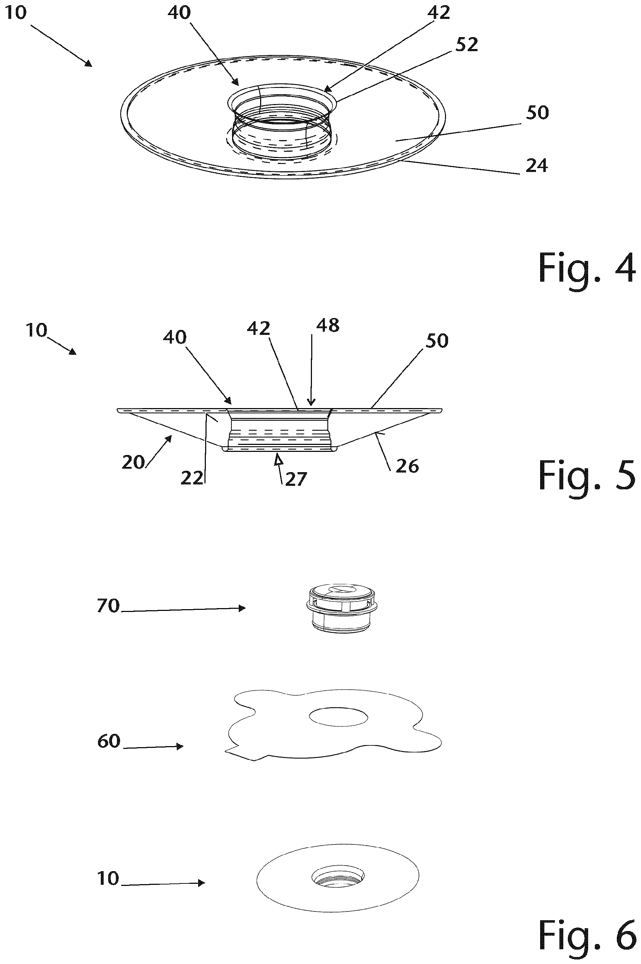

Fig. 4 und 5 zeigen eine weitere Ausgestaltung der Tracheostomaepithese 10, bei der die Abdeckplatte 50 einer distalen Seite des Aufnahmezylinders 42 zugeordnet ist. Fig. 4 zeigt die Tracheostomaepithese 10 in einer perspektivischen Ansicht. Fig. 5 zeigt die Tracheostomaepithese 10 in einer Seitenansicht. Der Aufnahmezylinder 42 ist im Wesentlichen im Epithesenkörper 20 versenkt beziehungsweise in der Atemöffnung 27 angeordnet. Die Abdeckplatte 50 erstreckt sich auf der distalen Seite 48 des Epithesenkörpers 20 von der distalen Außenkante 52 des Aufnahmezylinders 42 in Richtung eines Außenumfanges 24 des Epithesenkörpers 20. Auf diese Weise ist der Aufnahmezylinder 42 in der Atemöffnung 27 angeordnet und erstreckt sich in diesem Beispiel zumindest über die vollständige Höhe des Epithesenkörpers 20 durch diese.

Fig. 6 zeigt das System umfassend die Tracheostomaepithese 10 aus Fig. 4 und 5 sowie eines Pflasters 60 und eines Tracheostomahilfsmittels 70 in einer Explosionsansicht.

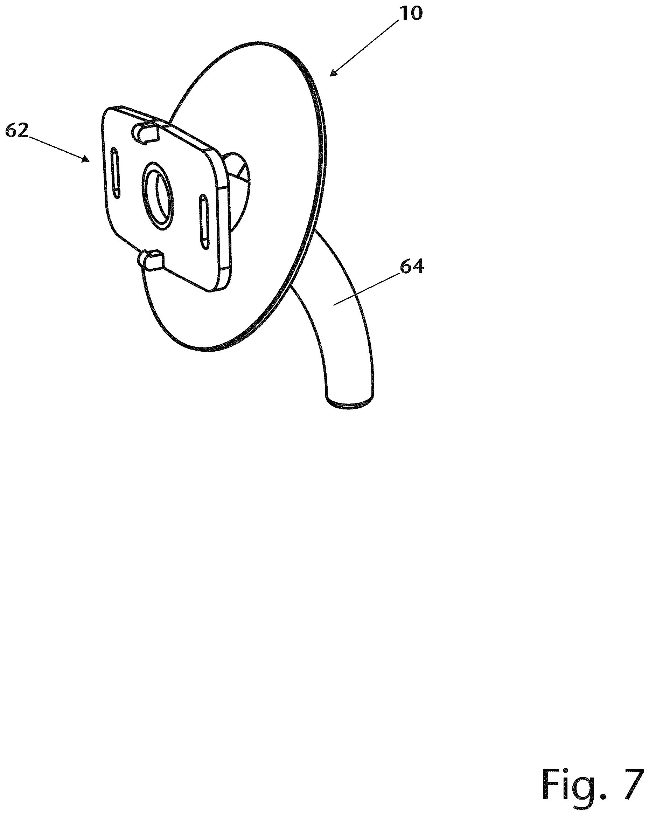

Fig. 7 ist die Tracheostomaepithese 10 aus Fig. 4 zu entnehmen, durch die ein Kanülenrohr 64 einer Trachealkanüle 62 geführt ist. Die Tracheostomaepithese 10 kann somit auch bei Verwendung einer Trachealkanüle 62 getragen werden.

Durch die vorgeschlagene Tracheostomaepithese 10 wird vorteilhafterweise Abfall eingespart und insbesondere können günstigere Pflaster zur Fixierung der Epithese verwendet werden. Weiterhin wird durch die Ausgestaltung der Tracheostomaepithese mit dem Aufnahmezylinder 42 in der Atemöffnung 27 eine kompakte Ausgestaltung vorgeschlagen, die komfortabler und ästhetisch ansprechender ist.

## Patentansprüche

1. Tracheostomaepithese (10) zumindest umfassend einen Epithesenkörper (20) und eine Aufnahmevorrichtung (40), wobei der Epithesenkörper (20) eine Grundform mit einer distalen Grundfläche (22) und einer proximalen Mantelfläche (26) aufweist, wobei der Epithesenkörper (20) eine Atemöffnung (27) umfasst, die sich durchgängig von proximal (p) nach distal (d) erstreckt, wobei die Aufnahmevorrichtung (40) einen Aufnahmezylinder (42) und eine Abdeckplatte (50) aufweist, wobei die Abdeckplatte (50) auf der Grundfläche (22) angeordnet ist und wobei die Abdeckplatte (50) mit dem Aufnahmezylinder (42) verbunden ist.

2. Tracheostomaepithese (10) nach Anspruch 1, **dadurch gekennzeichnet, dass** der Aufnahmezylinder (42) und die Abdeckplatte (50) materialeinheitlich sind.

3. Tracheostomaepithese (10) nach einem oder mehreren der vorhergehenden Ansprüche, **dadurch gekennzeichnet, dass** der Epithesenkörper (20) zumindest aus einem Material ist ausgewählt aus einer Gruppe umfassend Silikon und/oder selbstklebende Kunststoffe.

4. Tracheostomaepithese (10) nach einem oder mehreren der vorhergehenden Ansprüche, **dadurch gekennzeichnet, dass** das Material des Epithesenkörpers (20) ein selbstklebendes Material ist.

5. Tracheostomaepithese (10) nach einem oder mehreren der vorhergehenden Ansprüche, **dadurch gekennzeichnet, dass** die Aufnahmevorrichtung (40) aus zumindest einem Material ist ausgewählt aus einer Gruppe umfassend thermoplastische Elastomere.

6. Tracheostomaepithese (10) nach einem oder mehreren der vorhergehenden Ansprüche, **dadurch gekennzeichnet, dass** die Abdeckplatte (50) einer proximalen Seite (46) des Aufnahmezylinders (42) oder einer distalen Seite (48) des Aufnahmezylinders (42) zugeordnet ist.

7. Tracheostomaepithese (10) nach einem oder mehreren der vorhergehenden Ansprüche, **dadurch gekennzeichnet, dass** der Aufnahmezylinder (42) zumindest teilweise in der Atemöffnung (27) angeordnet ist.

8. Tracheostomaepithese (10) nach einem oder mehreren der vorhergehenden Ansprüche, **dadurch gekennzeichnet, dass** die Abdeckplatte (50) sich radial von dem Aufnahmezylinder (42) in Richtung eines Außenumfanges (24) des Epithesenkörpers (20) erstreckt.

9. Tracheostomaepithese (10) nach einem oder mehreren der vorhergehenden Ansprüche, **dadurch gekennzeichnet, dass** der Aufnahmezylinder (42) zumindest ein Rastmittel (44) zum Halten zumindest eines Tracheostomahilfsmittels (70) aufweist.

10. Verfahren zur Herstellung einer Tracheostomaepithese (10) nach einem oder mehreren der vorhergehenden Ansprüche, **dadurch gekennzeichnet, dass** diese im Mehr-Komponenten-Spritzguss hergestellt wird, wobei eine erste Komponente den Epithesenkörper (20) bildet und eine zweite Komponente die Aufnahmevorrichtung (40) bildet.

11. System zumindest umfassend eine Tracheostomaepithese (10) nach einem oder mehreren der Ansprüche 1 bis 9, ein Pflaster (60) und ein Tracheostomahilfsmittel (70), wobei das Pflaster (60) auf der Tracheostomaepithese (10) aufbringbar ist und das Tracheostomahilfsmittel (70) in eine Aufnahmevorrichtung (40) der Tracheostomaepithese (10) einbringbar ist.

## Claims

1. Tracheostoma epithesis (10) at least comprising an epithesis body (20) and a receiving device (40), the epithesis body (20) having a base shape with a distal base surface (22) and a proximal mantle surface (26), wherein the epithesis body (20) comprises a breathing opening (27) extending continuously from proximal (p) to distal (d), wherein the receiving device (40) comprises a receiving cylinder (42) and a cover plate (50), wherein the cover plate (50) is arranged on the base surface (22) and wherein the cover plate (50) is connected to the receiving cylinder (42).

2. Tracheostoma epithesis (10) according to claim 1, **characterized in that** the receiving cylinder (42) and the cover plate (50) are uniform in material.

3. Tracheostoma epithesis (10) according to one or more of the preceding claims, **characterized in that** the epithesis body (20) is made of at least one material selected from a group comprising silicone and/or self-adhesive plastics.

4. Tracheostoma epithesis (10) according to one or more of the preceding claims, **characterized in that** the material of the epithesis body (20) is a self-adhesive material.

5. Tracheostoma epithesis (10) according to one or more of the preceding claims, **characterized in that** the receiving device (40) is made of at least one material selected from a group comprising thermoplastic elastomers.

6. Tracheostoma epithesis (10) according to one or more of the preceding claims, **characterized in that** the cover plate (50) is associated with a proximal side (46) of the receiving cylinder (42) or a distal side (48) of the receiving cylinder (42).

7. Tracheostoma epithesis (10) according to one or more of the preceding claims, **characterized in that** the receiving cylinder (42) is arranged at least partially in the breathing opening (27).

8. Tracheostoma epithesis (10) according to one or more of the preceding claims, **characterized in that** the cover plate (50) extends radially from the receiving cylinder (42) towards an outer periphery (24) of the epithesis body (20).

9. Tracheostoma epithesis (10) according to one or more of the preceding claims, **characterized in that** the receiving cylinder (42) comprises at least one latching means (44) for holding at least one tracheostoma aid (70).

10. Method of manufacturing a tracheostoma epithesis (10) according to one or more of the preceding claims, **characterized in that** it is manufactured by multi-component injection molding, wherein a first component forms the epithesis body (20) and a second component forms the receiving device (40).

11. System comprising at least one tracheostoma epithesis (10) according to one or more of claims 1 to 9, a patch (60} and a tracheostoma aid (70), wherein the patch (60} is attachable to the tracheostoma epithesis (10) and the tracheostoma aid (70) is insertable into a receiving device (40) of the tracheostoma epithesis (10).

## Revendications

1. Épithèse trachéotomie (10) comprenant au moins un corps d'épithèse (20) et un dispositif de réception (40), ledit corps d'épithèse (20) présentant une forme de base avec une surface de base (22) distale et une surface d'enveloppe (26) proximale, ledit corps d'épithèse (20) comprenant une ouverture de respiration (27), qui s'étend de manière continue du côté proximal (p) au côté distal (d), ledit dispositif de réception (40) présentant un cylindre de réception (42) et une plaque de recouvrement (50), ladite plaque de recouvrement (50) étant disposée sur ladite surface de base (22) et ladite plaque de recouvrement (50) étant reliée audit cylindre de réception (42).

2. Épithèse trachéotomie (10) selon la revendication 1, **caractérisée en ce que** ledit cylindre de réception (42) et ladite plaque de recouvrement (50) sont constitués d'un seul matériau.

3. Épithèse trachéotomie (10) selon une ou plusieurs des revendications précédentes, **caractérisée en ce que** ledit corps d'épithèse (20) est constitué d'au moins un matériau choisi dans un groupe comprenant le silicone et/ou des matières plastiques auto-adhésives.

4. Épithèse trachéotomie (10) selon une ou plusieurs des revendications précédentes, **caractérisée en ce que** ledit matériau dudit corps d'épithèse (20) est un matériau auto-adhésif.

5. Épithèse trachéotomie (10) selon une ou plusieurs des revendications précédentes, **caractérisée en ce que** ledit dispositif de réception (40) est constitué d'au moins un matériau choisi dans un groupe comprenant des élastomères thermoplastiques.

6. Épithèse trachéotomie (10) selon une ou plusieurs des revendications précédentes, **caractérisée en ce que** ladite plaque de recouvrement (50) est associée à un côté (46) proximal dudit cylindre de réception (42) ou à un côté (48) distal dudit cylindre de réception (42).

7. Épithèse trachéotomie (10) selon une ou plusieurs des revendications précédentes, **caractérisée en ce que** ledit cylindre de réception (42) est disposé au moins partiellement dans ladite ouverture de respiration (27).

8. Épithèse trachéotomie (10) selon une ou plusieurs des revendications précédentes, **caractérisée en ce que** ladite plaque de recouvrement (50) s'étend radialement dudit cylindre de réception (42) vers une périphérie extérieure (24) dudit corps d'épithèse (20).

9. Épithèse trachéotomie (10) selon une ou plusieurs des revendications précédentes, **caractérisée en ce que** ledit cylindre de réception (42) présente au moins un moyen d'encliquetage (44) pour maintenir au moins un auxiliaire de trachéotomie (70).

10. Procédé de fabrication d'une épithèse de trachéotomie (10) selon une ou plusieurs des revendications précédentes, **caractérisé en ce que** celle-ci est fabriquée par moulage par injection à plusieurs composants, un premier composant formant ledit corps d'épithèse (20) et un deuxième composant formant ledit dispositif de réception (40).

11. Système comprenant au moins une épithèse de trachéotomie (10) selon l'une ou plusieurs des revendications 1 à 9, un pansement (60) et un auxiliaire de trachéotomie (70), dans lequel ledit pansement (60) peut être appliqué sur ladite épithèse de trachéotomie (10) et ledit auxiliaire de trachéotomie (70) peut être inséré dans un dispositif de réception (40) de ladite épithèse de trachéotomie (10).
